# EUROPEAN PATENT APPLICATION

(11) **EP 3 714 808 A2**
(43) Date of publication of application: **30.09.2020**
(21) Application number: 20165598.2
(22) Date of filing: 25.03.2020
(51) Int. Cl.: A61B 17/072, A61B 17/064, A61B 17/00

(54) **STAPLE CARTRIDGE AND STAPLES FOR SURGICAL STAPLING APPARATUS**

(30) Priority: 26.03.2019 US 201962824065 P; 18.02.2020 US 202016792974
(71) Applicant: Covidien LP, Mansfield, MA 02048 (US)
(72) Inventor: SWITALSKI, Christopher, Glastonbury, CT Connecticut 06033 (US); GADDY, Anthony, Windsor, CT Connecticut 06095 (US)
(74) Representative: Maschio, Antonio

(57) **Abstract**

A surgical stapling apparatus includes a handle assembly, an elongate tubular body extending distally from the handle assembly, and an end effector extending distally from the elongate tubular body. The end effector includes a staple cartridge assembly having a tissue facing surface defining retention slots and a central longitudinal slot. The retention slots are disposed in rows along the tissue facing surface, and each of the retention slots has an arcuate shape including a concave surface and a convex surface.

## Description

### CROSS-REFERENCE TO RELATED APPLICATIONS

This application claims the benefit of and priority to U.S. Provisional Patent Application Serial No. 62/824,065 filed March 26, 2019, the entire disclosure of which is incorporated by reference herein.

### BACKGROUND

### 1. Technical Field

The present application relates to surgical apparatus and more particularly, to end effectors, staple cartridges, and staples for use in surgical stapling apparatus.

### 2. Background of Related Art

Surgical stapling apparatus are employed by surgeons to sequentially or simultaneously apply one or more rows of fasteners, e.g., staples or two-part fasteners, to body tissue for the purpose of joining segments of body tissue together and/or creating anastomoses.

Linear surgical stapling apparatus generally include a pair of jaws or finger-like structures between which the body tissue to be joined is placed. Typically, the staples have straight backspans and are arranged in linear rows within retention slots defined in one of the jaws, with a gap or spacing between adjacent retention slots and adjacent rows. When the surgical stapling apparatus is actuated, or "fired", longitudinally moving firing bars or an actuation sled contact staple drive members in the jaw. The staple drive members push the staples through body tissue and into an anvil in the opposite jaw which forms the staples. If body tissue is to be removed or separated, a knife blade can be provided in one of the jaws of the surgical stapling apparatus to cut the body tissue between the lines of staples.

### SUMMARY

In an aspect of the present disclosure, a surgical stapling apparatus includes a handle assembly, an elongate tubular body extending distally from the handle assembly, and an end effector extending distally from the elongate tubular body. The end effector includes a staple cartridge assembly having a tissue facing surface defining retention slots and a central longitudinal slot. The retention slots are disposed in rows along the tissue facing surface, and each retention slot has an arcuate shape including a concave surface and a convex surface.

The retention slots in each row may be oriented in the same direction. The concave surfaces of the retention slots in adjacent rows may face each other.

In some aspects, a first row of retention slots is disposed adjacent to the central longitudinal slot and a second row of retention slots is disposed adjacent to the first row of retention slots such that the first row of retention slots is disposed between the central longitudinal slot and the second row of retention slots. A first pair of first and second rows of retention slots may be disposed on a first lateral side of the central longitudinal slot, and a second pair of first and second rows of retention slots may be disposed on a second lateral side of the central longitudinal slot. The convex surfaces of the retention slots of the first row of retention slots may face the central longitudinal slot, and/or the convex surfaces of the retention slots of the second row of retention slots may face an outer lateral edge of the tissue facing surface of the staple cartridge assembly.

In some aspects, adjacent rows of retention slots are longitudinal off-set with respect to each other. Each retention slot may include a central portion and end portions extending from opposed sides of the central portion, and the end portions of the retention slots in adjacent rows may be laterally opposed to each other. The central portion of each of the retention slots may be laterally aligned with a longitudinal space defined between the end portions of longitudinally adjacent retention slots of the adjacent row.

The concave and convex surfaces of the retention slots may each have a continuous and uniform curvature.

The end effector may include an anvil assembly having a tissue facing surface defining staple forming pockets and a central longitudinal slot formed therein, and the staple forming pockets may be complementary in shape and position with the retention slots of the staple cartridge assembly.

In some aspects, the end effector includes a number of staples corresponding to a number of the retention slots of the staple cartridge assembly. Each staple may include a backspan and legs extending from opposed ends of the backspan, and the staples may be shaped to correspond with the arcuate shape of the retention slots. The backspan of each staple may extend along a longitudinal axis with the ends of the backspan aligned along the longitudinal axis and a central portion of the backspan curved outwardly such that the central portion is laterally spaced from the longitudinal axis.

In another aspect of the present disclosure, an end effector includes a staple cartridge assembly and an anvil assembly. The staple cartridge assembly has a tissue facing surface defining retention slots and a central longitudinal slot. The retention slots are disposed in rows along the tissue facing surface, and each of the retention slots has an arcuate shape including a concave surface and a convex surface. The anvil assembly has a tissue facing surface defining staple forming pockets and a central longitudinal slot formed therein. The staple forming pockets are complementary in shape and position with the retention slots of the staple cartridge assembly.

In yet another aspect of the present disclosure, a staple cartridge assembly includes a staple cartridge and staples. The staple cartridge has a tissue facing surface defining retention slots and a knife slot. The retention slots are disposed in rows along the tissue facing surface, and each retention slot has an arcuate shape including a concave surface and a convex surface. The staples correspond in number to the retention slots of the staple cartridge. Each staple includes a backspan having an arcuate shape complementary to the arcuate shape of the retention slots.

The rows of the retention slots may be linear. The rows of the retention slots may be curved.

The tissue facing surface of the staple cartridge may include two rows of retention slots longitudinally off-set from each other. The retention slots in each of the two rows may be oriented in the same direction and the concave surface of the retention slots in each of the two rows may face each other.

Embodiments can include one or more of the following advantages.

In some embodiments, the staples are curved and are configured to create staple lines having larger staple coverage over the same area as compared to conventional straight line staples. The staples can have a longer backspan over the same distance as conventional staples to cover more area. Additionally or alternatively, the arced shape of the staples can allow for a tighter overlapping arrangement of the staples as compared to conventional staples. The design and/or layout of the staples create a more tortuous path for reducing the likelihood of tearing, leakage, and/or bleeding following medical stapling procedures.

In certain embodiments, the staples are configured for use with a curved or circular surgical stapling apparatus creating a more natural staple line with the curved or circular cut within tissue or a tubular body lumen.

### BRIEF DESCRIPTION OF THE DRAWINGS

The above and other aspects, features, and advantages of the present disclosure will be apparent in light of the following detailed description when taken in conjunction with the accompanying drawings, which are incorporated in and constitute a part of this specification, wherein:
FIG. 1 is a perspective view of a surgical stapling apparatus having an end effector in accordance with an embodiment of the present disclosure;
FIG. 2 is an exploded, perspective view of staple cartridge and anvil assemblies of the end effector of the surgical stapling apparatus of FIG. 1;
FIG. 3 is a top view of a tissue facing surface of the staple cartridge assembly of FIG. 2;
FIG. 4 is a perspective view of the end effector of the surgical stapling apparatus of FIG. 1, showing a tissue facing surface of a staple cartridge assembly of the end effector;
FIG. 5 is a perspective view of the end effector of the surgical stapling apparatus of FIG. 1, showing a tissue facing surface of an anvil assembly of the end effector;
FIG. 6 is a perspective view of a staple, shown in an initial configuration, for use within a staple cartridge assembly of the end effector of FIG. 1;
FIG. 7 is a perspective view of the staple of FIG. 6, shown in a formed configuration;
FIG. 8 is a top view of tissue that has been divided and stapled with the surgical stapling apparatus of FIG. 1; and
FIGS. 9A-9C are perspective views of end effectors in accordance with other embodiments of the present disclosure.

### DETAILED DESCRIPTION OF EMBODIMENTS

Embodiments of the present disclosure will now be described in detail with reference to the drawing figures wherein like reference numerals identify similar or identical elements. Throughout this description, the term "proximal" refers to a portion of a structure, or component thereof, that is closer to a user, and the term "distal" refers to a portion of the structure, or component thereof, that is farther from the user.

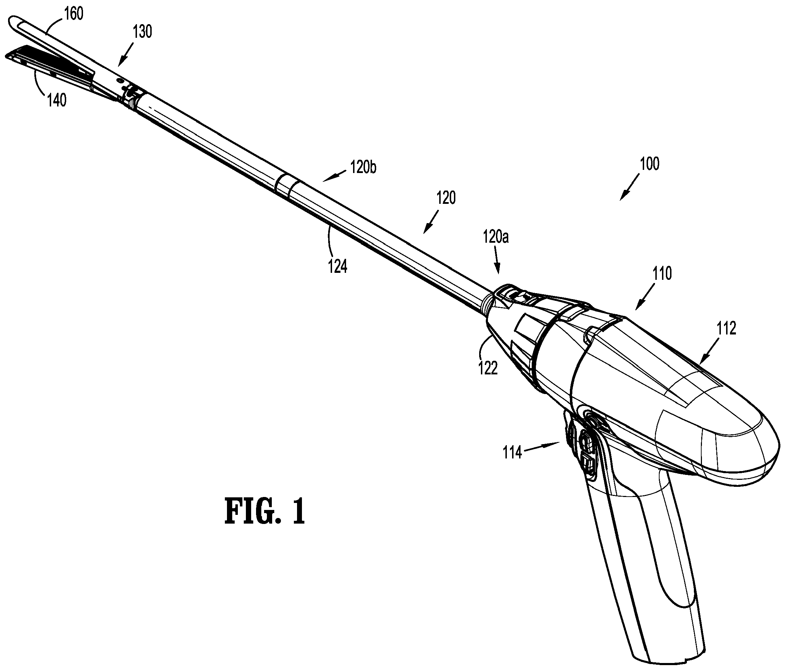

Referring now to FIG. 1, a surgical stapling apparatus or device 100 is shown in the form of a powered handheld electromechanical surgical instrument. The surgical stapling apparatus 100 includes a powered handle assembly 110, an adapter assembly 120, and a tool assembly or end effector 130. The powered handle assembly 110 is configured for selective connection with the adapter assembly 120 and, in turn, the adapter assembly 120 is configured for selective connection with the end effector 130.

The surgical stapling apparatus 100 will only further be described to the extent necessary to disclose aspects of the present disclosure. For a detailed description of the structure and function of exemplary surgical apparatus, and components thereof, reference may be made to commonly owned U.S. Patent Publication Nos. 2016/0296234 and 2016/0310134, and U.S. Patent Application No. 15/972,606, the entire contents of each of which are incorporated herein by reference.

With continued reference to FIG. 1, the powered handle assembly 110 includes a handle housing 112 housing a power-pack (not shown) configured to power and control various operations of the surgical stapling apparatus 100, and a plurality of actuators 114 (e.g., finger-actuated control buttons, knobs, toggles, slides, interfaces, and the like) for activating various functions of the surgical stapling apparatus 100. The adapter assembly 120 has a proximal portion 120a including a knob housing 122 configured for operable connection to the handle assembly 110 and a distal portion 120b including an outer tube or elongate tubular body 124 configured for operable connection to the end effector 130. The end effector 130 includes a loading unit or staple cartridge assembly 140 and an anvil assembly 160.

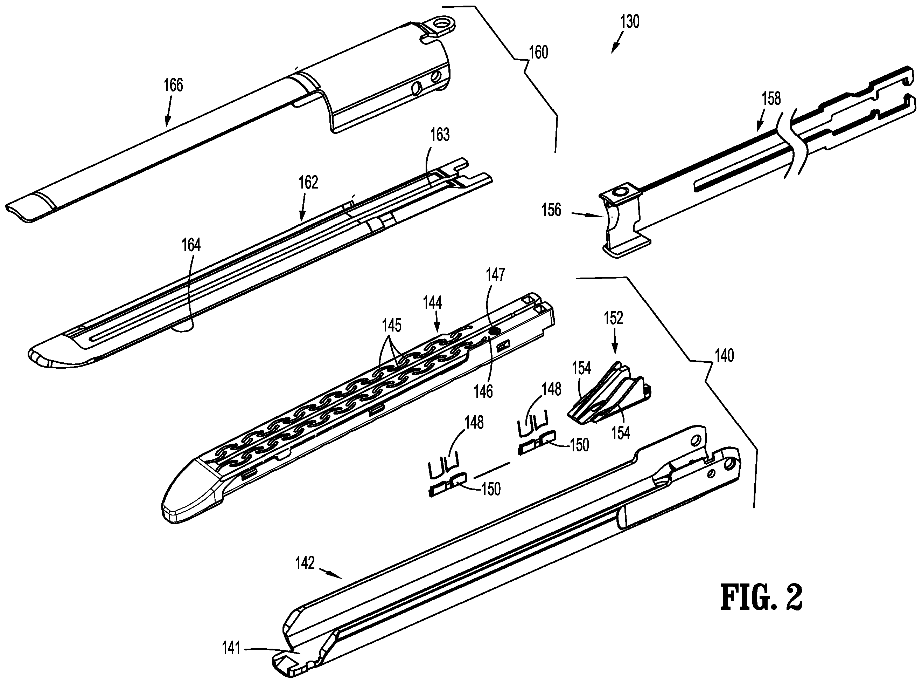

As shown in FIG. 2, the staple cartridge assembly 140 of the end effector 130 includes a cartridge carrier 142 defining an elongated support channel 141 configured and dimensioned to selectively receive a staple cartridge 144 therein. The staple cartridge 144 is removable and replaceable in the cartridge carrier 142 of the staple cartridge assembly 140. The staple cartridge 144 includes a tissue facing or contacting surface 146 defining retention slots 145 formed therein. A plurality of fasteners or staples 148 and staple pushers 150 are disposed in staple pockets (not explicitly shown) for engagement with an actuation sled 152 for ejection of the staples 148 through the retention slots 145. The actuation sled 152 is translatable through the staple cartridge 144 to advance cam wedges 154 of the actuation sled 152 into sequential contact with the staple pushers 150. A central longitudinal or knife slot 147 is formed in and extends along a substantial length of the staple cartridge 144 to facilitate passage of a knife blade 156 of a drive bar 158 therethrough.

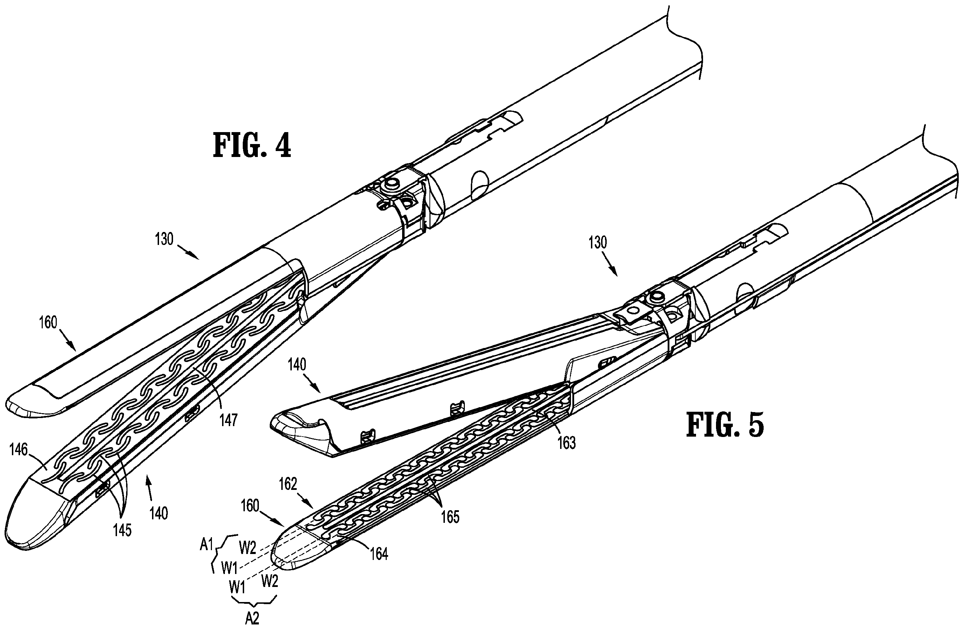

The anvil assembly 160 includes an anvil plate 162 having a tissue facing or contacting surface 164 defining a central longitudinal slot 163 formed therein, and a cover plate 166 secured over the anvil plate 162. The anvil plate 162 may include staple forming pockets or cavities 165 (FIG. 5) defined in the tissue facing surface 164 thereof.

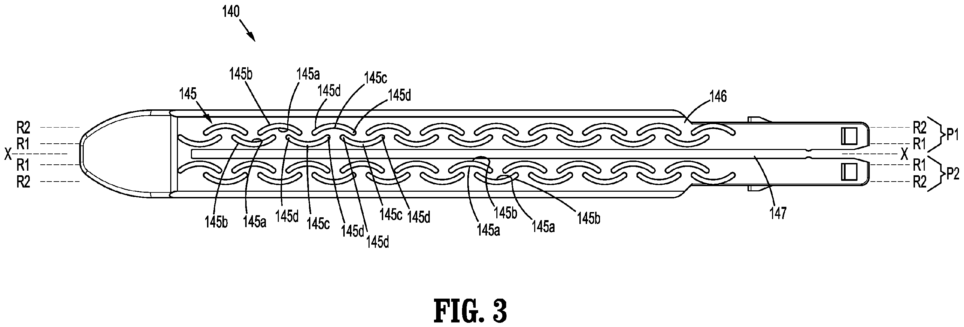

As shown in FIGS. 3 and 4, the retention slots 145 of the staple cartridge assembly 140 are disposed in rows along the longitudinal length of the tissue facing surface 146 of the staple cartridge assembly 140. An inner or first row "R1" of retention slots 145 is disposed adjacent to the central longitudinal slot 147 and an outer or second row "R2" of retention slots 145 is disposed adjacent the first row "R1" such that the first row "R1" is disposed between the central longitudinal slot 147 and the second row "R2." The first and second rows "R1," "R2" of retention slots 145 are defined on each side of the central longitudinal slot 147 such that a first pair "P1" of first and second rows "R1," "R2" is disposed on a first lateral side of the central longitudinal slot 147 and a second pair "P2" of first and second rows "R1," "R2" is disposed on a second lateral side of the central longitudinal slot 147. It is envisioned that additional rows of retention slots may be incorporated into the tissue facing surface, and such additional rows may have the same or a different configuration from the first and/or second rows.

Each retention slot 145 has an arcuate or bowed shaped having a concave surface 145a and a convex surface 145b. The concave and convex surfaces 145a, 145b each has a continuous and uniform curvature throughout its entire surface. Each retention slot 145 further includes a central portion 145c and end portions 145d extending from opposed sides of the central portion 145c. The retention slots 145 in each of the first and second rows "R1," "R2" are oriented in the same direction, with the first and second rows "Rl," "R2" disposed in opposite orientations relative to each other such that the concave surfaces 145a of the retention slots 145 in each of the first and second rows "R1," "R2" face each other in overlapping relation. The first and second rows "R1," "R2" are longitudinally off-set with respect to each other such that the central portion 145c of each retention slot 145 is laterally aligned with a longitudinal space defined between the end portions 145d of adjacent retention slots 145 of the opposed row, and the end portions 145d of the retention slots 145 in each of the first and second rows "R1," "R2" overlap and are laterally opposed to each other. It should be understood that the longitudinal and/or lateral spacing between the retention slots 145 may be adjusted to change the coverage over the staple area. For example, the end portions of adjacent retention slots may be positioned within the central portion of an opposed retention slot for increased staple coverage.

As shown, the first rows "R1" of the retention slots 145 are configured such that the convex surfaces 145b are disposed adjacent to and face the central longitudinal slot 147 of the tissue facing surface 146 and the end portions 145d extend laterally therefrom towards the second row "R2." The second rows "R2" of the retention slots 145 are configured such that the concave surface 145a faces the central longitudinal slot 146.

With reference now to FIG. 5, the staple forming pockets 165 of the anvil assembly 160 are disposed in rows along the longitudinal length of the tissue facing surface 164 of the anvil plate 162. The geometry and orientation of the staple forming pockets 165 correspond with that of the retention slots 145 of the staple cartridge assembly 140. Specifically, an inner or first row "W1" of staple forming pockets 165 is disposed adjacent to the central longitudinal slot 163 and an outer or second row "W2" of staple forming pockets 165 is disposed adjacent the first row "W1" such that the first row "W1" is disposed between the central longitudinal slot 153 and the second row "W2." The first and second rows "W1," "W2" of staple forming pockets 165 are defined on each side of the central longitudinal slot 163 such that a first pair "A1" of the first and second rows "W1," "W2" is disposed on a first lateral side of the central longitudinal slot 163 and a second pair "A2" of the first and second rows "W1," "W2" is disposed on a second lateral side of the central longitudinal slot 163. It is envisioned that additional rows of staple forming pockets may be incorporated into the tissue facing surface, and such additional rows may have the same or a different configuration from the first and/or second rows.

Each staple forming pocket 165 has a shape corresponding with the shape of the retention slots 145 (FIG. 4) of the staple cartridge assembly 140. The shape of the staple forming pockets 165 may be larger than the retention slots 145 such that the receiving area is expanded to form and clinch the staples 148 (FIG. 2).

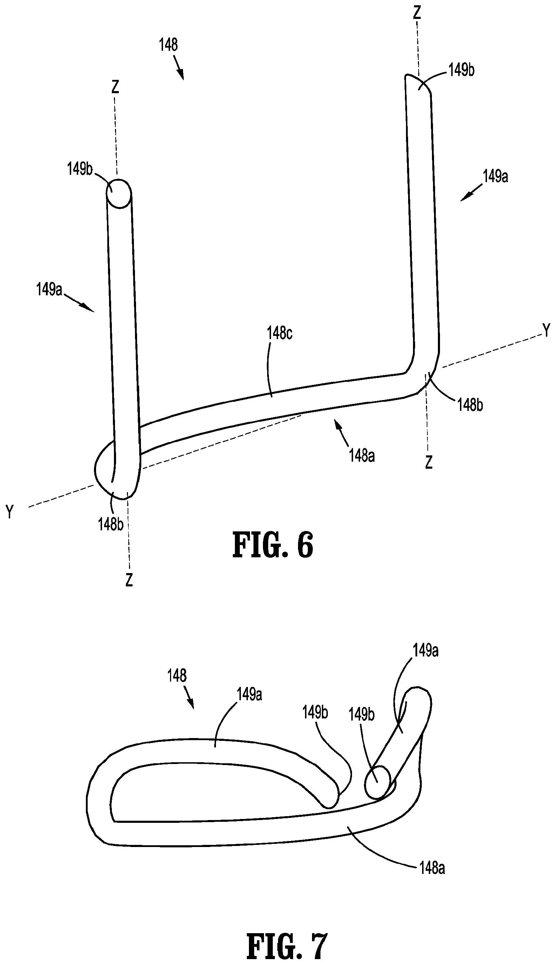

As shown in FIG. 6, the staples 148, disposed in the retention slots 145 (see e.g., FIG. 4) of the staple cartridge assembly 140, include a backspan 148a and a pair of legs 149a extending from opposed ends 148b of the backspan 148a and terminating in tissue penetrating tips 149b. The staples 148 are shaped to correspond with the shape of the retention slots 145. The backspan 148a of each staple 148 extends along a longitudinal axis "Y" with the ends 148b of the backspan 148a aligned along the longitudinal axis "Y" and a central portion 148c of the backspan 148a curved outwardly therefrom such that the central portion 148c is laterally spaced from the longitudinal axis "Y." The pair of legs 149a extend along a transverse axis "Z" that is perpendicular to the longitudinal axis "Y".

In use, the surgical stapling apparatus 100 is actuated by movement of one of the plurality of actuators 114 (FIG. 1) relative to powered handle assembly 110 (e.g., a trigger). The drive bar 158 of the staple cartridge assembly 140 moves distally thereby causing the anvil assembly 160 to be moved from an open position, spaced from the staple cartridge assembly 140, to a closed position relative to the staple cartridge assembly 140 to capture tissue therebetween. As the drive bar 158 is advanced distally within the staple cartridge 144, the actuation sled 152 translates through the staple cartridge 144 to advance the cam wedges 154 into sequential contact with the staple pushers 150, to cause the staple pushers 150 to translate vertically within the retention slots 145 and urge the staples 148 from the retention slots 145 towards the anvil plate 162 of the anvil assembly 160. The tissue penetrating tips 149b of the pair of legs 149a of the staples 148 contact the staple forming pockets 165 of the anvil assembly 160 such that the pair of legs 149b are bent towards the backspan 148a, as shown in FIG. 7, to form the staples 148 and secure the tissue.

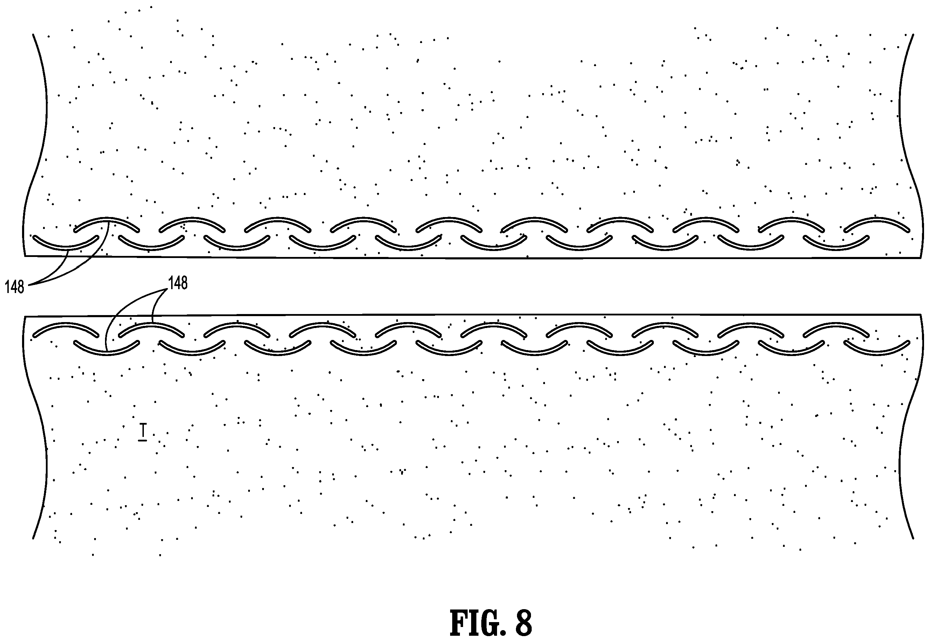

Upon full actuation of the surgical stapling apparatus 100, the knife blade 156, which is carried by the drive bar 158, cuts the tissue disposed between the rows of now formed staples 148. Upon movement of the anvil assembly 160 back to the open position, the resultant tissue "T" is divided and stapled closed with the staples 148, is illustrated in FIG. 8. The shape and configuration of the staples 148 within the tissue "T" cover more of the stapled area as compared to conventional straight staples and create a tortuous path within the stapled area having regions of overlap for reducing potential leaks.

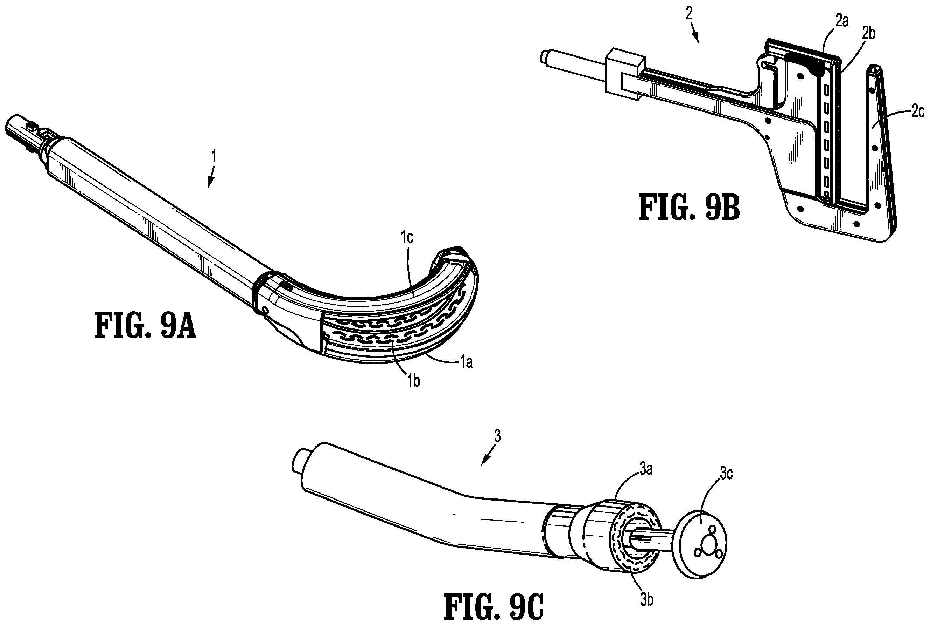

It should be understood that the end effectors, staple cartridge assemblies, and/or staples described herein may be configured for use with other surgical apparatus, such as: manual surgical stapling apparatus as described, for example, in U.S. Patent Nos. 8,256,656, 7,819,896, and 7,128,253; open staplers as described, for example, in U.S. Patent No. 7,334,717; endoscopic staplers having radial or curved reloads as described, for example, in U.S. Patent No. 8,360,298; transverse anastomosis staplers as described, for example, in U.S. Patent No. 5,964,394; end-to-end anastomosis staplers having circular staple cartridge and anvil assemblies as described, for example, in U.S. Patent Nos. 4,473,077, 5,119,983, and 5,915,616; as well as robotic surgical systems as described, for example, in U.S. Patent No. 8,828,023, the entire content of each of which is incorporated herein by reference. For example, as shown in FIGS. 9A-9C, end effectors 1-3, respectively, suitable for use with the surgical stapling apparatus 100 of FIG. 1 may include staple cartridge assemblies 1a-3a having at least two rows of retention slots 1b-3c (e.g., curved or annular rows with regard to staplers 1, 3 and linear rows with regard to stapler 2) retaining staples (not shown) therein, and associated anvil assembly 1c-3c, similar to those described above with respect to end effector 130. It should be further understood that the end effectors and/or staple cartridge assemblies may house surgical fasteners other than staples having the disclosed configuration.

While embodiments of the disclosure have been shown in the drawings, it is not intended that the disclosure be limited thereto, as it is intended that the disclosure be as broad in scope as the art will allow and that the specification be read likewise. It is to be understood, therefore, that the present disclosure is not limited to the precise embodiments described, and that various other changes and modifications may be effected by one skilled in the art without departing from the scope or spirit of the disclosure. Additionally, the elements and features shown and described in connection with certain embodiments may be combined with the elements and features of certain other embodiments without departing from the scope of the present disclosure, and that such modifications and variation are also included within the scope of the present disclosure. Therefore, the above description should not be construed as limiting, but merely as exemplifications of preferred embodiments. Thus the scope of the embodiments should be determined by the appended claims and their legal equivalents, rather than by the examples given.

The invention may be described by reference to the following numbered paragraphs:-
1. A surgical stapling apparatus comprising:
   a handle assembly;
   an elongate tubular body extending distally from the handle assembly; and
   an end effector extending distally from the elongate tubular body, the end effector including a staple cartridge assembly having a tissue facing surface defining retention slots and a central longitudinal slot, the retention slots disposed in rows along the tissue facing surface, each of the retention slots having an arcuate shape including a concave surface and a convex surface.
2. The surgical stapling apparatus of paragraph 1, wherein the retention slots in each of the rows are oriented in the same direction.
3. The surgical stapling apparatus of paragraph 2, wherein the concave surfaces of the retention slots in adjacent rows face each other.
4. The surgical stapling apparatus of paragraph 1, wherein a first row of the rows of the retention slots is disposed adjacent to the central longitudinal slot and a second row of the rows of the retention slots is disposed adjacent to the first row such that the first row is disposed between the central longitudinal slot and the second row.
5. The surgical stapling apparatus of paragraph 4, wherein a first pair of first and second rows is disposed on a first lateral side of the central longitudinal slot, and a second pair of first and second rows is disposed on a second lateral side of the central longitudinal slot.
6. The surgical stapling apparatus of paragraph 4, wherein the convex surfaces of the retention slots of the first row faces the central longitudinal slot.
7. The surgical stapling apparatus of paragraph 6, wherein the convex surfaces of the retention slots of the second row faces an outer lateral edge of the tissue facing surface of the staple cartridge assembly.
8. The surgical stapling apparatus of paragraph 1, wherein adjacent rows of the retention slots are longitudinal off-set with respect to each other.
9. The surgical stapling apparatus of paragraph 8, wherein each of the retention slots includes a central portion and end portions extending from opposed sides of the central portion, and the end portions of the retention slots in adjacent rows are laterally opposed to each other.
10. The surgical stapling apparatus of paragraph 9, wherein the central portion of each of the retention slots is laterally aligned with a longitudinal space defined between the end portions of longitudinally adjacent retention slots of the adjacent row.
11. The surgical stapling apparatus of paragraph 1, wherein the concave and convex surfaces of the retention slots each has a continuous and uniform curvature.
12. The surgical stapling apparatus of paragraph 1, wherein the end effector includes an anvil assembly having a tissue facing surface defining staple forming pockets and a central longitudinal slot formed therein, the staple forming pockets are complementary in shape and position with the retention slots of the staple cartridge assembly.
13. The surgical stapling apparatus of paragraph 1, wherein the end effector includes a number of staples corresponding to a number of the retention slots of the staple cartridge assembly.
14. The surgical stapling apparatus of paragraph 13, wherein each of the staples includes a backspan and legs extending from opposed ends of the backspan, the staples shaped to correspond with the arcuate shape of the retention slots.
15. The surgical stapling apparatus of paragraph 14, wherein the backspan of each of the staples extends along a longitudinal axis with the ends of the backspan aligned along the longitudinal axis and a central portion of the backspan curved outwardly such that the central portion is laterally spaced from the longitudinal axis.
16. An end effector comprising:
   a staple cartridge assembly having a tissue facing surface defining retention slots and a central longitudinal slot, the retention slots disposed in rows along the tissue facing surface, each of the retention slots having an arcuate shape including a concave surface and a convex surface; and
   an anvil assembly having a tissue facing surface defining staple forming pockets and a central longitudinal slot formed therein, the staple forming pockets are complementary in shape and position with the retention slots of the staple cartridge assembly.
17. A staple cartridge assembly comprising:
   a staple cartridge having a tissue facing surface defining retention slots and a knife slot, the retention slots disposed in rows along the tissue facing surface, each of the retention slots having an arcuate shape including a concave surface and a convex surface; and
   staples corresponding in number to the retention slots of the staple cartridge, each of the staples including a backspan having an arcuate shape complementary to the arcuate shape of the retention slots.
18. The staple cartridge assembly of paragraph 17, wherein the rows of the retention slots are linear.
19. The staple cartridge assembly of paragraph 17, wherein the rows of retention slots are curved.
20. The staple cartridge assembly of paragraph 17, wherein the staple cartridge includes two rows of retention slots longitudinally off-set from each other, the retention slots in each of the two rows oriented in the same direction and the concave surface of the retention slots in each of the two rows facing each other.

## Claims

1. A surgical stapling apparatus comprising:
a handle assembly;
an elongate tubular body extending distally from the handle assembly; and
an end effector extending distally from the elongate tubular body, the end effector including a staple cartridge assembly having a tissue facing surface defining retention slots and a central longitudinal slot, the retention slots disposed in rows along the tissue facing surface, each of the retention slots having an arcuate shape including a concave surface and a convex surface.

2. The surgical stapling apparatus of claim 1, wherein the retention slots in each of the rows are oriented in the same direction.

3. The surgical stapling apparatus of claim 2, wherein the concave surfaces of the retention slots in adjacent rows face each other.

4. The surgical stapling apparatus of any preceding claim, wherein a first row of the rows of the retention slots is disposed adjacent to the central longitudinal slot and a second row of the rows of the retention slots is disposed adjacent to the first row such that the first row is disposed between the central longitudinal slot and the second row.

5. The surgical stapling apparatus of claim 4, wherein a first pair of first and second rows is disposed on a first lateral side of the central longitudinal slot, and a second pair of first and second rows is disposed on a second lateral side of the central longitudinal slot; and/or wherein the convex surfaces of the retention slots of the first row faces the central longitudinal slot; preferably wherein the convex surfaces of the retention slots of the second row faces an outer lateral edge of the tissue facing surface of the staple cartridge assembly.

6. The surgical stapling apparatus of any preceding claim, wherein adjacent rows of the retention slots are longitudinal off-set with respect to each other; preferably wherein each of the retention slots includes a central portion and end portions extending from opposed sides of the central portion, and the end portions of the retention slots in adjacent rows are laterally opposed to each other; preferably still wherein the central portion of each of the retention slots is laterally aligned with a longitudinal space defined between the end portions of longitudinally adjacent retention slots of the adjacent row.

7. The surgical stapling apparatus of any preceding claim, wherein the concave and convex surfaces of the retention slots each has a continuous and uniform curvature.

8. The surgical stapling apparatus of any preceding claim, wherein the end effector includes an anvil assembly having a tissue facing surface defining staple forming pockets and a central longitudinal slot formed therein, the staple forming pockets are complementary in shape and position with the retention slots of the staple cartridge assembly.

9. The surgical stapling apparatus of any preceding claim, wherein the end effector includes a number of staples corresponding to a number of the retention slots of the staple cartridge assembly; preferably wherein each of the staples includes a backspan and legs extending from opposed ends of the backspan, the staples shaped to correspond with the arcuate shape of the retention slots.

10. The surgical stapling apparatus of claim 9, wherein the backspan of each of the staples extends along a longitudinal axis with the ends of the backspan aligned along the longitudinal axis and a central portion of the backspan curved outwardly such that the central portion is laterally spaced from the longitudinal axis.

11. An end effector comprising:
a staple cartridge assembly having a tissue facing surface defining retention slots and a central longitudinal slot, the retention slots disposed in rows along the tissue facing surface, each of the retention slots having an arcuate shape including a concave surface and a convex surface; and
an anvil assembly having a tissue facing surface defining staple forming pockets and a central longitudinal slot formed therein, the staple forming pockets are complementary in shape and position with the retention slots of the staple cartridge assembly.

12. A staple cartridge assembly comprising:
a staple cartridge having a tissue facing surface defining retention slots and a knife slot, the retention slots disposed in rows along the tissue facing surface, each of the retention slots having an arcuate shape including a concave surface and a convex surface; and
staples corresponding in number to the retention slots of the staple cartridge, each of the staples including a backspan having an arcuate shape complementary to the arcuate shape of the retention slots.

13. The staple cartridge assembly of claim 12, wherein the rows of the retention slots are linear.

14. The staple cartridge assembly of claim 12, wherein the rows of retention slots are curved.

15. The staple cartridge assembly of claim 12, wherein the staple cartridge includes two rows of retention slots longitudinally off-set from each other, the retention slots in each of the two rows oriented in the same direction and the concave surface of the retention slots in each of the two rows facing each other.
